# FASCICULE DE BREVET EUROPEEN

(11) **EP 0 526 370 B1**
(45) Date de publication et mention de la délivrance du brevet: **11.03.1998**
(21) Numéro de dépôt: 92450011.9
(22) Date de dépôt: 23.07.1992
(51) Int. Cl.: G01N 35/10, G01N 35/02

(54) **Appareil d'analyse automatique d'échantillons**
Automatisches Analysegerät für Proben
Automatic analyser of samples

(30) Priorité: 26.07.1991 FR 9109833
(43) Date de publication de la demande: 03.02.1993
(73) Titulaire: SOCIETE FRANCAISE DE RECHERCHES ET D'INVESTISSEMENTS (SFRI),Société Anonyme, 33127 Martignas sur Jalle (FR)
(72) Inventeur: Rafilipomanana, Christiane, F-33600 Pessac (FR); Coeurveille, Michel, F-33127 Martignas sur Jalle (FR); Barach, Yves, F-33700 Mérignac (FR); Millet, Magali, F-33700 Salles (FR)
(74) Mandataire: Thébault, Jean-Louis

(56) Documents cités:
- EP-A- 0 351 988
- WO-A-87/05401
- DE-A- 3 346 532
- DE-A- 3 441 179
- GB-A- 2 190 999
- US-A- 3 772 154

## Description

Cette invention concerne un appareil qui permet d'effectuer automatiquement une ou plusieurs analyses sur différents échantillons afin de déterminer la présence ou la quantité d'une ou plusieurs substances contenues dans les échantillons. Il permet, plus particulièrement, d'effectuer des essais (ou des tests) d'immunologie sur les liquides biologiques tels que le sérum ou le plasma, ou d'effectuer des tests de biochimie.

Dans le domaine de l'immunologie, les tests sont effectués en général avec une plaque de microtitration contenant 96 supports de réaction ou puits répartis en 12 colonnes de 8 puits. Il est bien connu que les différentes étapes d'un test d'immunologie sur plaque de microtitration peuvent être faites soit manuellement, soit à l'aide d'appareils automatiques, mais les appareils connus n'automatisent qu'une partie des différentes étapes. Par exemple, il existe des appareils qui automatisent les manipulations de liquides tels que les sérums et les réactifs, mais ils ne gérent pas les temps d'incubation et n'assurent pas les opérations de lavage des puits, ni les lectures à la fin des réactions. L'un des inconvénients d'effectuer les tests manuellement, ou à l'aide de ces appareils, est la disparité des temps d'incubation entre les différents puits d'où des erreurs dans les résultats. Un autre inconvénient est la monopolisation d'un technicien, soit pour effectuer les opérations manuelles, soit pour transférer les plaques d'un appareil à un autre, par exemple transférer d'un manipulateur automatique de liquides à un incubateur, puis à un laveur de plaques, puis à nouveau à un manipulateur automatique de liquide, puis à un incubateur, puis à un lecteur de plaques de 96 puits.

Le brevet européen 0351 988 mentionne un appareil qui automatise les différentes étapes des tests d'immunologie, mais cet appareil assure une gestion par plaque de 96 puits et non par puits. L'inconvénient est de ne pouvoir mélanger sur la plaque divers tests d'immunologie ayant des temps d'incubation, entre les étapes, différents.

Le manque de souplesse, de rapidité, de précision, ainsi que le risque d'erreurs dues aux manipulations nous ont conduit à concevoir un appareil entièrement automatique, objet de cette invention.

Il est bien connu qu'il existe aussi des appareils automatiques pour effectuer les étapes d'un essai d'immunologie sur des supports de réaction spécifiques à l'appareil et non sur des supports généralement utilisés tels que les puits d'une plaque de microtitration. L'inconvénient est l'obligation de n'utiliser que les supports de réactions spécifiques à l'appareil. De plus, avec ces appareils, l'utilisateur ne peut effectuer en même temps qu'un seul type d'analyse par échantillon et doit tranférer manuellement les échantillons, contenus dans leur tube, vers les supports de réactions spécifiques à l'appareil.

Le but de l'invention est précisément de rémédier à ces inconvénients.

Par le document DE-A-3 346 532, on connaît un instrument d'analyses chimiques dans lequel les échantillons à doser sont acheminés sous les stations de traitement par bandes transporteuses, les plaques supports de cuvettes coniques de réaction étant mobiles par un système d'ascenseur. Toutefois, un tel appareil n'est pas complètement autonome et automatique et n'est pas en fait véritablement polyvalent.

Le document WO-A-87/05401 décrit un appareil d'analyse automatique d'échantillons conforme au préambule de la revendication 1. Cependant, cet appareil présente les mêmes inconvénients que ceux cités pour le document EP-A-0 351 988 concernant l'incapacité d'une gestion par puits.

Le but de l'invention est de remédier aux inconvénients des dispositifs rappelés ci-dessus.

A cet effet, l'invention a pour objet un appareil d'analyse automatique d'échantillons comprenant :
- au moins une tête mobile au-dessus d'un plan de travail comportant une zone où sont disposés les échantillons à analyser, une zone où sont disposés les réactifs, une zone où sont disposés les puits de réaction et une zone comprenant au moins un système de lecture pour mesurer les résultats d'analyse,
- au moins un premier sous-ensemble de prélèvement et de distribution de liquides, ledit premier sous-ensemble étant porté par ladite tête et comprenant un chariot mobile verticalement et portant au moins un tube connecté à au moins un tuyau relié à un système d'aspiration-refoulement monté sur le bâti de l'appareil,
- un système mécanique de déplacement de ladite tête dans un plan horizontal suivant deux directions,
- des moyens pour réguler thermiquement lesdits puits de réaction,
- et des moyens de commande, contrôle et coordination,
caractérisé :
- en ce qu'il comporte en outre une zone munie d'au moins un bol de lavage,
- en ce que lesdites zones comportant les échantillons, les réactifs, les puits de réaction, le système de lecture et le bol de lavage sont accolées et statiques dans un plan unique constituant ledit plan de travail,
- en ce que ladite tête dessert l'ensemble desdites zones,
- et en ce que lesdits moyens de commande, contrôle et coordination sont agencés de façon à agir, d'une part, sur ladite tête en sorte qu'elle puisse se déplacer au-dessus de chacune desdites zones pour prélever et transférer un liquide d'une zone à une autre ou pour effectuer le lavage dudit tube dans ledit bol de lavage, d'autre part, sur lesdits moyens de régulation thermique et, enfin, sur ledit système de lecture.

D'autres caractéristiques et avantages concernant la constitution et le fonctionnement des divers éléments et sous-ensembles de l'appareil ressortiront de la description détaillée qui va suivre d'un mode de réalisation dudit appareil, description donnée à titre d'exemple uniquement et en regard des dessins annexés sur lesquels :
La figure 1 représente en perspective un appareil automatique selon l'invention ;
La figure 2 est une vue de côté en élévation de l'appareil dessiné en figure 1 avec arrachement d'une partie de l'appareil.
La figure 3 est une vue de face de la tête, la figure 4 une vue de gauche de la tête et la figure 5, une vue de droite.
La figure 6 est une vue en plan de dessus de l'appareil dessiné en figure 1 avec arrachement d'une partie de l'appareil pour montrer le plan de travail.
La figure 7 est une vue en coupe partielle du bol de lavage.
La figure 8 est une vue schématique d'un système de lecture par mesure de la quantité de lumière absorbée.
La figure 9 est une vue schématique, en coupe, du système de régulation thermique des cuves de réactions.
La figure 10 est une vue schématique du système mécanique qui sert à déplacer la tête.
La figure 11 schématise le système informatique.

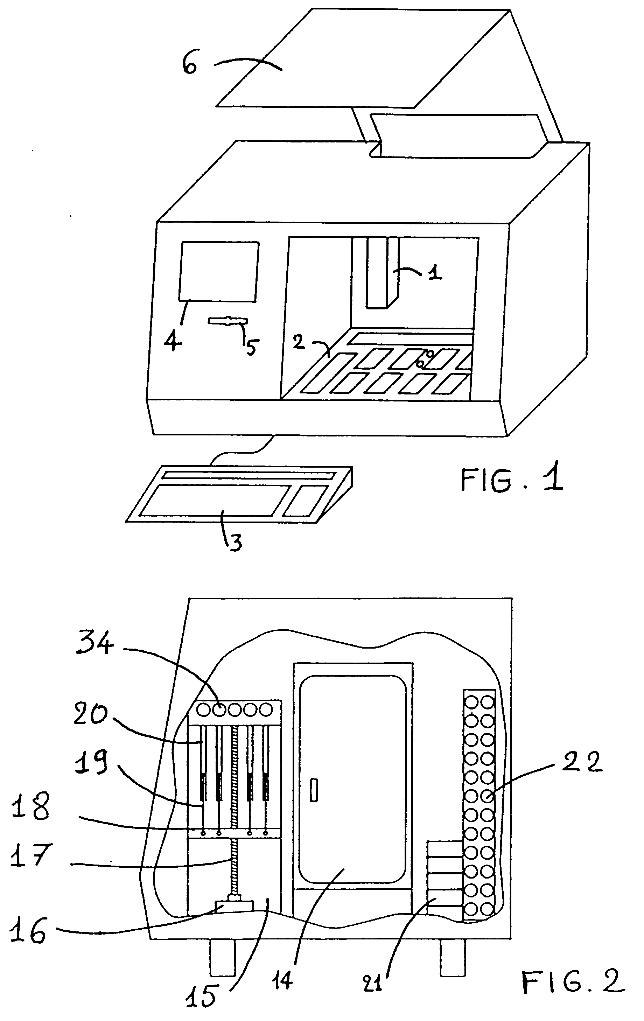

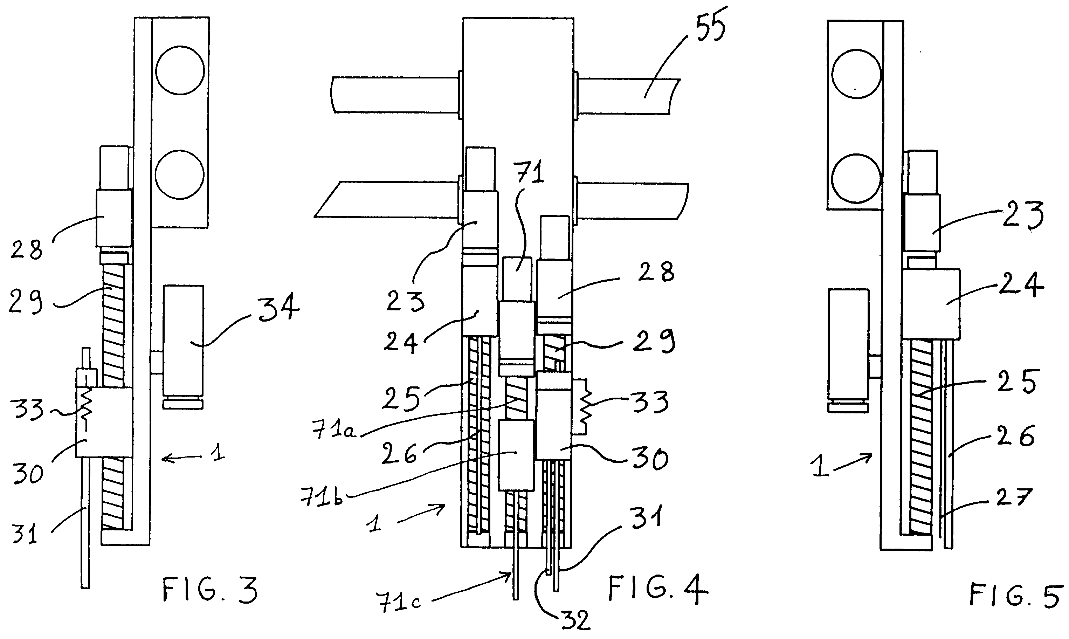

On a représenté en figures 1 et 2 l'appareil automatique d'analyse d'échantillons pour montrer les principaux éléments. Certains sous-ensembles sont représentés plus en détail dans les figures 3 à 11.

Sur la figure 1, on a représenté l'appareil avec une seule tête (1). Pour augmenter les cadences d'analyse, on pourrait installer d'autres têtes.

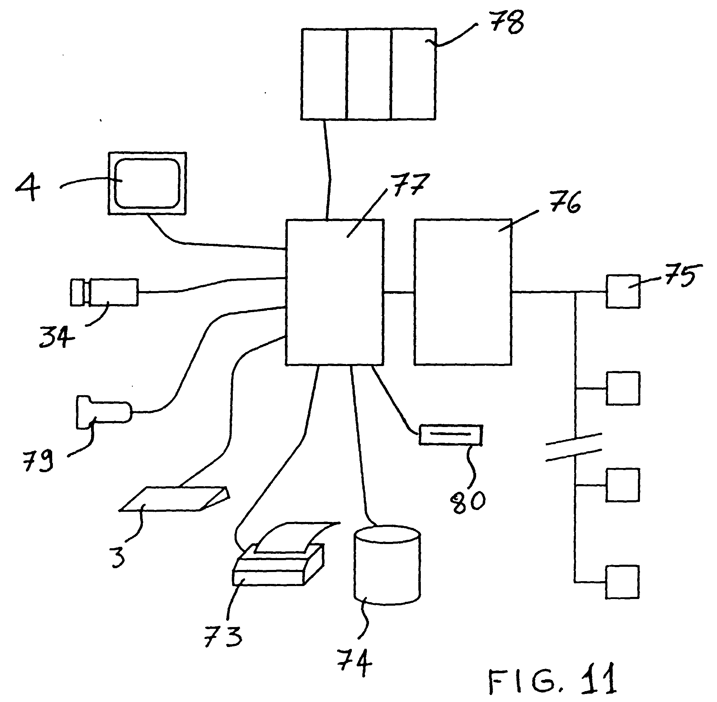

Sur la figure 1, on peut voir la tête (1), qui se déplace au dessus du plan de travail (2). Le clavier (3) permet d'identifier les échantillons et de communiquer avec un premier ordinateur (cf. fig 11), qui lui-même échange des informations avec un deuxième ordinateur qui pilote les microprocesseurs de commande des moteurs ou des entrées-sorties logiques. Dans le mode de réalisation illustré, les des ordinateurs sont intégés à l'appareil. Le lecteur de disquette (5) permet d'entrer des programmes, d'introduire des protocoles ou de recevoir des résultats. Le moniteur couleur (4) permet de visualiser des informations. Lorsque le système est en fonctionnement, la porte (6) est fermée et est bloquée électromagnétiquement pour des raisons de sécurité.

Sur le plan de travail représenté en figure (6), on range les échantillons à analyser en positions (7) ou (8). Les réactifs spécifiques à l'analyse d'un paramètre sont disposés en zone (9) et les réactifs communs à plusieurs analyses, comme par exemple les solutions de dilutions des échantillons, sont rangés en zone (10) ou dans la boîte réfrigérée par effet Peltier représentée en (14) sur la figure 2.

Les supports de réaction ou puits, qui sont conventionnels, sont disposés sur quatre plaques, par exemple de 96 puits de réaction chacune. Une plaque est représentée en (11). La zone (9) est réfrigérée par effet Peltier et peut être agitée pour homogénéiser les réactifs.

La tête (1) qui se déplace au dessus du plan de travail est réprésentée en figures 3, 4 et 5 et comprend, dans le mode de réalisation représenté, trois sous-ensembles. L'un des sous-ensembles comprend un moteur (23) qui actionne une vis sans fin (25) qui fait déplacer un chariot (24). Sur le chariot, on a fixé une pipette ou un petit tube (26), et, en parallèle, un fil conducteur (27) pour détecter la surface du liquide à aspirer. Un deuxième sous-ensemble comprend le moteur (28) qui actionne la vis sans fin (29) qui déplace le chariot (30). Sur ce chariot est fixé au moins un ensemble de deux petits tubes. L'un des tubes (31) permet d'aspirer le liquide contenu dans un support de réaction. Ce tube (31) est rappelé vers le chariot par le ressort (33). Le chariot (30) descend suffisamment pour que le tube (31) touche le fond du support de réaction et que le ressort (33), en se tendant, maintienne bien le tube contre le fond tout en amortissant le choc au moment du contact du tube avec le fond. L'autre tube (32) sert à l'envoi d'une solution de lavage dans les puits. La prévision de plusieurs ensembles 28 à 32 permet de laver en même temps plusieurs supports de réaction.

Sur la tête, on a fixé également une caméra de type CCD (34). Si la profondeur de champ de la caméra est suffisante, elle peut être solidaire de la tête. C'est ce qui est représenté sur la figure 3 ou la figure 5. Sinon, elle peut être solidaire d'un chariot mû verticalement par une vis sans fin comme est mû le chariot (24) ou le chariot (30).

Cette caméra peut permettre d'analyser une étiquette qui identifie un tube contenant l'échantillon ou qui identifie un réactif ou une boîte contenant plusieurs réactifs, ou qui identifie un support de réaction. Les étiquettes peuvent être codifiées avec des barres. La caméra peut vérifier la position sur le plan de travail des différents éléments : tubes échantillons, réactifs, supports de réaction et contrôler ainsi une erreur éventuelle faite par l'opérateur lors du chargement du plan de travail. Ainsi, les échantillons, les réactifs ou les supports de réaction peuvent être disposés sur le plan de travail dans des positions appropriées mais indifférenciées par l'opérateur, d'où l'élimination de tous risques d'erreurs par celui-ci. De plus, si un bouchon, par exemple a été oublié par l'opérateur sur un tube ou un flacon contenant un réactif, celui-ci sera analysé par la caméra et le système informatique enverra un message à l'opérateur et l'analyse ne commencera qu'après que le bouchon ait été enlevé. Cette caméra peut aussi, par analyse d'image, interpréter le résultat d'une réaction, par exemple elle peut permettre de mesurer une agglutination de complexes immuns traduisant la présence d'un analyte (anticorps ou antigène) dans l'échantillon. Elle permet aussi de repérer la position de la tête au dessus du plan de travail.

Pour distribuer ou aspirer avec précision des volumes d'échantillons ou de réactifs compris entre 1 µl et 500 µl, le système mécanique est représenté en figure 2. Un moteur (16) actionne une vis sans fin (17) qui fait mouvoir verticalement le chariot (18). Ce chariot est solidaire de quatre pistons (19) de seringues (20). L'une des extrémités des seringues est solidaire du bâti mécanique (15). Pour distribuer des volumes, on peut aussi utiliser des pompes (21). Un ensemble d'électrovannes (34) et (22) permettent d'orienter les liquides dans les différents tuyaux. Les petits tubes 26, 31 et 32, sont reliés, par des tuyaux non représentés, aux diverses électrovannes 22, 34.

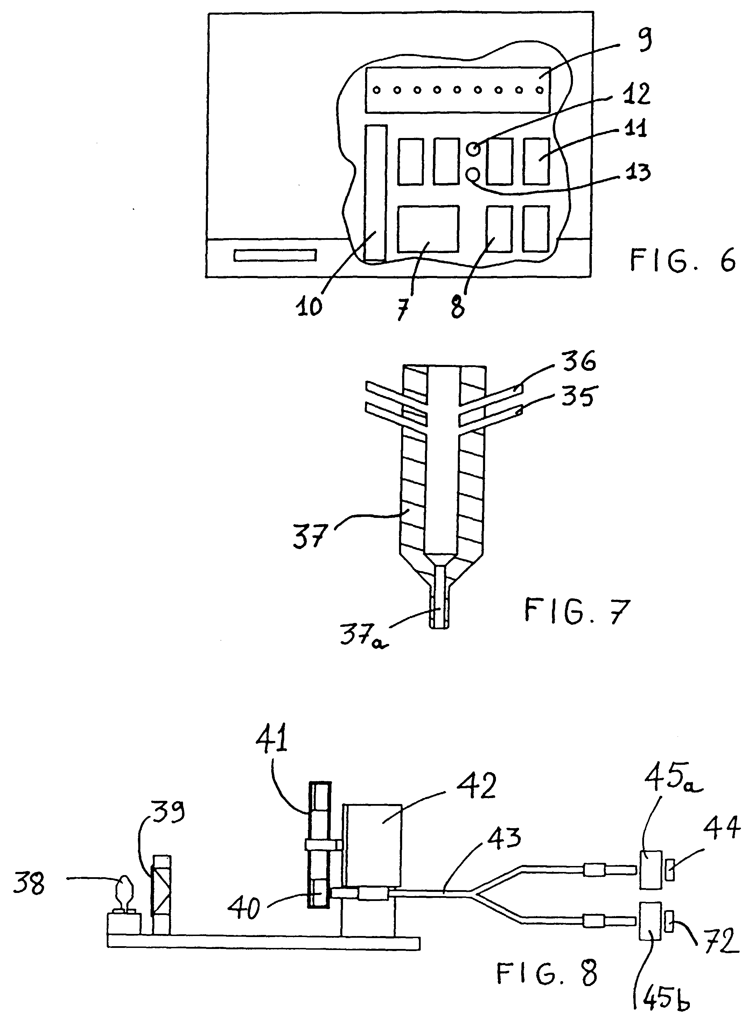

Lorsque le petit tube (26) a aspiré, puis distribué à l'aide du système mécanique (15), un volume d'échantillon par exemple, il va être lavé en venant se positionner dans le bol de lavage (37) représenté sur la figure 7 et en (12) sur le plan de travail représenté sur la figure 6. La tête va donc se déplacer au dessus du bol de lavage, le moteur (23) va faire tourner la vis sans fin (25) qui va faire descendre le chariot (24) sur lequel sont fixés le petit tube (26) et le fil (27). Le petit tube et le fil vont rentrer dans le bol (37). Pour laver l'intérieur du petit tube (26), de l'eau déminéralisée ou une solution de lavage puis de l'eau déminéralisée va arriver par le tuyau connecté en haut du petit tube (26), circuler dans le petit tube et être rejeté dans le bol (37) qui se vide par l'orifice (37a) à l'aide d'une pompe (non représentée). Pour laver l'extérieur du petit tube (26) et le fil (27), une solution de lavage puis de l'eau déminéralisée sont injectées par des buses (35), au nombre par exemple de 6. Pour éviter la formation de gouttelettes sur le petit tube ou entre le petit tube (26) et le fil (27), de l'air est injecté à travers les buses (36).

La figure 8 schématise le système de lecture qui permet de mesurer la quantité de lumière absorbée par le liquide réactionnel. Une lampe (38) émet à travers un condenseur (39) et un filtre (40) fixé sur une tourelle (41). Cette tourelle, qui porte dix filtres différents, est mue par un moteur (42). Après le filtre (40), la lumière optique passe à travers une fibre optique (43) en forme de Y pour envoyer la lumière vers 2 cellules ou photodiodes (44, 72) après être passé à travers 2 cuves dont l'une (45a) contient l'échantillon à mesurer, l'échantillon ayant été amené dans le petit tube (26) à travers l'orifice (13) sur le plan de travail.

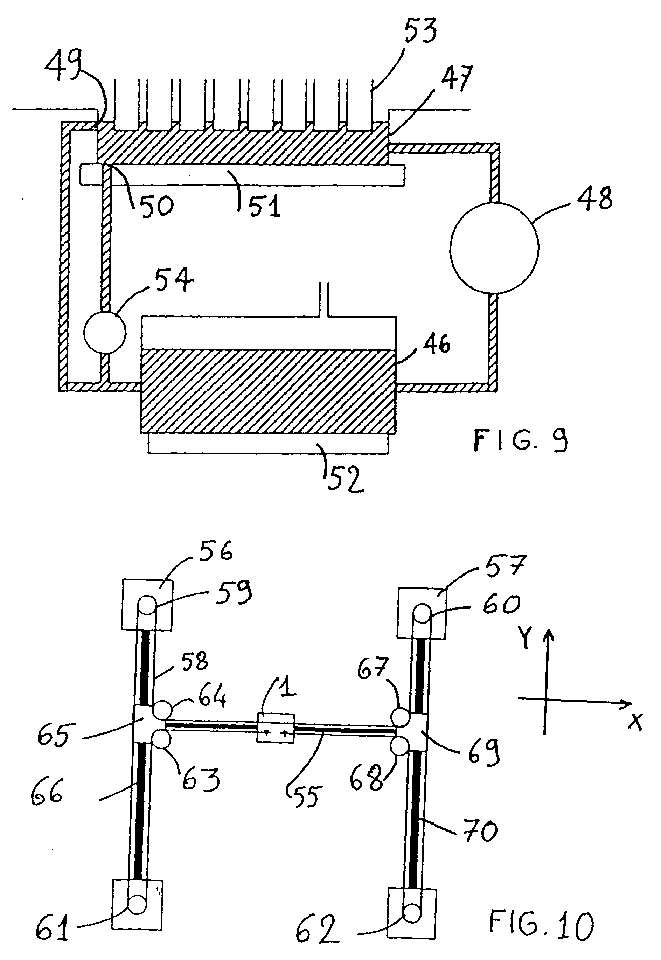

La figure 9 schématise le système de régulation thermique des supports de réaction (53). Le réservoir (46) contient un liquide qui est mis en circulation vers le bac (47) par une pompe (48). Le trop plein du bac est évacué par l'orifice (49). Le liquide baigne le fond ou une partie des supports de réaction suivant la hauteur de l'orifice (49). La plaque (51), dont la température est contrôlée, permet de chauffer le liquide dont la température est également mesurée. La plaque (52) est refroidie par effet Peltier et permet d'abaisser la température du liquide. On pourrait aussi utiliser l'effet Peltier pour chauffer la plaque (52). A la fin des analyses, on vide le bac (47) par l'orifice (50) en actionnant l'électrovanne (54).

La tête (1) se déplace horizontalement suivant les axes X et Y grâce au système mécanique représenté sur la figure 10. Des moteurs (56) et (57) sont fixes et entraînent par un ensemble de poulies une courroie (58) solidaire de la tête (1). Les deux poulies (59) et (60) sont solidaires de l'axe des moteurs. Les poulies (61) et (62) sont fixes. Les poulies (63) et (64) sont solidaires du chariot (65) qui se déplace sur la barre (66). Les poulies (67) et (68) sont solidaires du chariot (69) qui se déplace sur la barre (70). La tête (1) se déplace sur les barres (55). Les deux extrémités de la courroie (58) sont fixées sur la tête (1). Le déplacement de la tête est contrôlé par le sens de rotation des moteurs et le nombre de tours que chacun effectue.

L'identification des échantillons, des réactifs et des supports de réaction peut se faire soit au clavier (3), soit à l'aide d'un lecteur code barres (79), ou à l'aide de la caméra CCD (34). Les protocoles des analyses sont saisis soit au clavier (3), soit sont rentrés par le lecteur de disquettes (80) pour être mémorisé sur le disque dur (74). Les résultats des analyses sont édités sur l'imprimante (73), sont mémorisés sur le disque dur (74) et peuvent être transmis vers un ordinateur central extérieur (78). Les entrées et sorties décrites ci-dessus sont gérées par l'ordinateur (77). L'ordinateur (77) communique avec l'ordinateur (76) qui pilote les micro-processeurs (75). Ces microprocesseurs gèrent les moteurs et les entrées-sorties logiques telles que l'ouverture et la fermeture de la porte (6).

Les deux ordinateurs (76) et (77) peuvent être extérieurs à l'automate. Un seul ordinateur pourrait être utilisé mais le système informatique serait moins performant.

A l'aide d'un exemple et en se référant aux dessins, on va exposer un mode de fontionnement de l'appareil automatique.

Avec le clavier (3), on saisit l'identification des sérums à analyser : le nom du patient, son numéro et les analyses souhaitées pour un échantillon, par exemple dosage de la ferritine pour le premier échantillon.

Les tubes de sérum de diamètre 13 mm (hauteur : 74 mm) sont positionnés dans un rack qui est rangé en position (7) sur le plan de travail. Les tubes de diamètre 9 mm (hauteur : 42 mm) sont positionnés dans un ou deux racks rangés en position (8).

Après identification des sérums, le système informatique, par l'un des ordinateurs, regroupe les analyses et indique comment positionner les puits de réaction des les plaques à 96 positions et comment ranger les plaques sur les quatre positions (11). Le système s'assure ensuite que les réactifs propres à chaque analyse ont bien été positionnés en zone (9), sinon il indique à l'utilisateur, à l'aide du moniteur couleur (4), l'emplacement en zone (9), où un coffret de réactifs doit être rajouté et demande à l'utilisateur d'identifier ce coffret à l'aide du clavier (3) en indiquant le nom du paramètre et le numéro de lot.

Le système organise les séquences des analyses avec un logiciel approprié pour optimiser ses déplacements puis il commence les analyses suivant les protocoles préalablement mémorisés sur le disque dur.

Les moteurs (56) et (57) entraînent la courroie (59) qui déplace les barres (55) suivant l'axe Y et qui déplace la tête (1) suivant l'axe X. La tête se positionne au dessus du premier tube d'échantillon, le moteur (23) actionne la vis sans fin (25) qui déplace vers le bas le chariot (24) auquel sont solidaires le petit tube (26) et le fil (27) jusqu'à ce que ceux-ci rencontrent la surface de l'échantillon liquide. Le moteur (16) actionne la vis sans fin (17) qui déplace le chariot (18) vers le bas entraînant les 4 seringues. L'une des quatre seringues a un volume de 500 µl et les trois autres de 2500 µl. Par le jeu des électrovannes, le piston de la seringue de 500 µl en se déplaçant, crée une dépression dans le petit tube (26) pour aspirer par exemple 20 µl d'échantillon. Le moteur (23) permet de remonter le chariot (24). La tête est déplacée au dessus du puits de réaction (53) relatif à la première analyse de l'échantillon. Le petit tube (26) est descendu, le piston de la seringue de 500 µl est remonté par le chariot (18) de façon à ce que les 20 µl de l'échantillon soient distribués dans le puits de réaction (53) dont les parois sont recouvertes d'anticorps anti-ferritine. Ensuite, la tête est positionnée au dessus du bol de lavage représenté sur la figure 7. Le petit tube (26) et le fil (27) sont descendus. Par le jeu d'électrovannes, la pompe (21) permet d'envoyer une solution de lavage puis de l'eau déminéralisée à travers le petit tube (26), ces solutions étant évacuées du bol de lavage à travers l'orifice (37). En même temps, à travers six buses (35), des jets de solution de lavage puis d'eau déminéralisée sont envoyés sur l'extérieur du petit tube. Au bout de quelques secondes, quatre de préférence, à travers deux buses (36), un jet d'air empêche la formation de gouttelettes sur le tube. Enfin, le chariot (24) remonte, un autre cycle peut se répéter pour amener dans le puits de réaction 100 µl de réactif constitué par exemple par un anticorps anti-ferritine conjugué à une enzyme telle que la horseradish peroxydase.

Pendant 30 minutes, on laisse les 20 µl de sérum incuber avec les 100 µl d'anticorps anti-ferritine conjugué à la peroxydase en présence de l'anticorps anti-ferritine complémentaire fixé sur les parois du puits. Pendant ce temps, la tête peut continuer à transférer des sérums et des anticorps conjugués dans d'autres puits. Au bout de 30 minutes, sous le contrôle du système informatique, la tête revient se positionner au dessus du puits (53).

Le moteur (28) actionne la vis sans fin (29) pour descendre le chariot (33) jusqu'à ce que le tube (31) touche le fond du puits et que le contact soit fermement maintenu par le ressort (33). Par le tube (31), à l'aide d'une pompe (non représentée), le liquide contenu dans le puits est aspiré, puis par le tube (32) à l'aide d'une autre pompe, une solution de lavage est distribuée dans le puits. Ce cycle peut être répété plusieurs fois. On termine par une aspiration du contenu du puits. Après ce lavage de puits, la tête se déplace légèrement, un moteur (71) déplace par une vis sans fin (71a) un chariot (71b) portant un ensemble (71c) de petits tubes. A travers l'un d'eux, on distribue 100 µl d'un premier réactif et à travers un deuxième tube, on distribue 100 µl d'un deuxième réactif. Ces deux réactifs sont le substrat de l'enzyme. On laisse incuber ces deux réactifs pendant 15 minutes. Pendant ce temps la tête peut répéter les cycles ci-dessus sur d'autres puits.

Au bout de 15 minutes, le liquide s'est coloré dans le puits, la tête vient se positionner à nouveau au dessus du puits et à l'aide du petit tube (26) 200 µl de liquide sont aspirés. La tête se déplace au dessus de la cuve de lecture (45a) située sous l'orifice (13) et distribue 50 µl qui sont aussitôt aspirés à l'aide d'une pompe par l'orifice situé au fond de la cuve. Ceci afin de chasser les résidus de l'échantillon précédent qui pourraient fausser la lecture par une modification de la couleur. On répète l'opération avec 50 µl de plus. Puis, on distribue les 100 µl restants. La lecture se fait sur la cellule (44) à travers les 100 µl du liquide réactionnel (cuve 45a) et sur l'autre cellule (72) à travers un liquide de référence (cuve 45b). On évacue les 100 µl de liquide réactionnnel, on les remplace par le liquide de référence et on refait les mesures sur les cellules (44) et (72) à travers les liquides de référence. Ainsi, on peut tenir compte du vieillissement des cuves, en particulier de celle (45a) qui reçoit le liquide réactionnel. Le résultat obtenu est affiché sur l'écran (4), est imprimé sur l'imprimante (73), est mémorisé sur le disque dur (74). Si l'appareil automatique est connecté à un ordinateur central (78), celui-ci peut aussi mémoriser les résultats pour un traitement ultérieur.

L'appareil selon l'invention est particulièrement destiné aux essais d'immunologie en phase homogène ou en phase hétérogène pour la recherche ou le dosage d'un analyte, appelé anticorps ou antigène, dans un liquide biologique. Il est aussi destiné au dosage de l'ADN - Acide Désoxyribo Nucléique, présent en position extracellulaire dans le sérum.

L'exemple indiqué ci-dessus ne limite pas l'invention à cette réalisation. L'invention englobe aussi toutes autres variantes constructives.

C'est ainsi que les sous-ensembles composant la tête (1) peuvent être répétés huit fois par exemple pour une gestion par huit supports de réaction à la fois, au lieu d'un. Dans ce cas, le bol de lavage (37) et le ou les systèmes de lecture seront également au nombre de huit.

## Revendications

1. Appareil d'analyse automatique d'échantillons comprenant :
• au moins une tête (1) mobile au-dessus d'un plan de travail (2) comportant une zone (7, 8) où sont disposés les échantillons à analyser, une zone (9, 10) où sont disposés les réactifs, une zone (11) où sont disposés les puits de réaction (53) et une zone (13) comprenant au moins un système (45a) de lecture pour mesurer les résultats d'analyse,
• au moins un premier sous-ensemble (23 à 26) de prélèvement et de distribution de liquides, ledit premier sous-ensemble étant porté par ladite tête et comprenant un chariot (26) mobile verticalement et portant au moins un tube (26) connecté à au moins un tuyau relié à un système d'aspiration-refoulement (15) monté sur le bâti de l'appareil,
• un système mécanique (55 à 70) de déplacement de ladite tête dans un plan horizontal suivant deux directions,
• des moyens (46 à 52) pour réguler thermiquement lesdits puits de réaction (53),
• et des moyens (3,4,34 et 73 à 80) de commande, contrôle et coordination,
caractérisé :
• en ce qu'il comporte en outre une zone (12) munie d'au moins un bol de lavage (37),
• en ce que lesdites zones (7 à 13) comportant les échantillons, les réactifs, les puits de réaction (53), le système de lecture (45a) et le bol de lavage (37) sont accolées et statiques dans un plan unique constituant ledit plan de travail (2),
• en ce que ladite tête (1) dessert l'ensemble desdites zones (7 à 13),
• et en ce que lesdits moyens (3, 4, 34 et 73 à 80) de commande, contrôle et coordination sont agencés de façon à agir, d'une part, sur ladite tête (1) en sorte qu'elle puisse se déplacer au-dessus de chacune desdites zones (7 à 13) pour prélever et transférer un liquide d'une zone à une autre ou pour effectuer le lavage dudit tube (26) dans ledit bol de lavage (37), d'autre part, sur lesdits moyens de régulation thermique (46 à 52) et, enfin, sur ledit système de lecture (45a).

2. Appareil suivant la revendication 1, caractérisé en ce que ladite tête (1) comporte au moins un second sous-ensemble (30 à 32) destiné au lavage des puits de réaction (53).

3. Appareil suivant la revendication 2, caractérisé en ce que ledit second sous-ensemble comporte un chariot (30) sur lequel est fixé au moins un tube (31) relié à au moins un tuyau, pour l'aspiration du liquide contenu dans un puits de réaction (53) et au moins un tube (32) relié à au moins un tuyau, pour la distribution d'une solution dans un puits de réaction (53), lesdits tuyaux étant reliés audit système d'aspiration-refoulement (15).

4. Appareil suivant la revendication 3, caractérisé en ce qu'il comporte des moyens pour fixer lesdits tubes (31, 32) audit chariot (30) en sorte d'assurer un bon contact avec le fond d'un puits de réaction (53) tout en compensant les éventuelles différences de hauteur entre les fonds desdits puits de réaction.

5. Appareil suivant la revendication 4, caractérisé en ce que lesdits moyens de fixation sont un ressort (13).

6. Appareil suivant l'une quelconque des revendications 1 à 5, caractérisé en ce que ledit bol de lavage (37) comporte un orifice inférieur (37a) par lequel il peut être vidé et au moins une buse (35) d'injection de liquide pour nettoyer l'extérieur dudit tube (26) lorsque celui-ci est partiellement introduit dans ledit bol (37).

7. Appareil suivant la revendication 6, caractérisé en ce que ledit bol de lavage (37) comporte en outre au moins une buse (36) d'injection d'air pour sécher l'extérieur dudit tube (26) lorsque ce dernier est partiellement introduit dans ledit bol.

8. Appareil suivant la revendication 1, caractérisé en ce que lesdits moyens (46 à 52) de régulation thermique utilisent un liquide de régulation thermique en contact au moins partiel avec lesdits puits (53).

9. Appareil suivant l'une quelconque des revendications 1 à 8, caractérisé en ce que ladite tête (1) comporte au moins un troisième sous-ensemble constitué d'une caméra (34) d'analyse du bon positionnement de la tête et/ou des codes d'identification des échantillons et/ou des réactifs et de lecture des résultats d'analyse.

10. Appareil suivant la revendication 9, caractérisé en ce que ladite caméra (34) est montée fixe sur ladite tête (1).

11. Appareil suivant la revendication 10, caractérisé en ce que ladite caméra (34) est montée mobile verticalement sur ladite tête (1).

12. Appareil suivant l'une quelconque des revendications 1 à 11, caractérisé en ce que ledit système de lecture comprend :
• un premier récipient (45b) contenant un liquide de référence ;
• un second récipient (45a) contenant un échantillon à mesurer ;
• une fibre optique (43) en forme de Y envoyant la lumière vers lesdits premier et second récipients (45b, 45a), et
• des moyens optiques (72, 44) pour recevoir la lumière provenant desdits premier et second récipients.

13. Appareil suivant la revendication 12, caractérisé en ce que lesdits moyens optiques comprennent une photodiode ou cellule (72, 44) associée à chacun desdits premier et second récipients (45b, 45a).

14. Appareil suivant l'une quelconque des revendications 1 à 13, caractérisé en ce que lesdits moyens pour commander, contrôler et coordonner comprennent des moyens informatiques (3, 4, 34, 73 à 80) aptes à mémoriser les protocoles des analyses, gérer l'identification des échantillons, des réactifs et des puits de réaction (53), gérer les différentes étapes des analyses, gérer les résultats des analyses et aider l'utilisateur dans le fonctionnement et la maintenance de l'appareil.

## Patentansprüche

1. Automatisches Probenanalysegerät, umfassend:
- mindestens einen über einer Arbeitsebene (2) beweglichen Kopf (1), welche Arbeitsebene eine Zone (7, 8), in der die zu analysierenden Proben angeordnet sind, eine Zone (9, 10), in der die Reagentien angeordnet sind, eine Zone (11), in der die Reaktionsvertiefungen (53) angeordnet sind, und eine Zone (13) mit mindestens einem Ablesesystem (45a) für das Messen der Analyseresultate umfaßt,
- mindestens eine erste Baugruppe (23 bis 26) für Entnahme und Verteilung von Flüssigkeiten, welche erste Baugruppe von dem Kopf abgestützt ist und einen vertikalbeweglichen Schlitten (26) umfaßt, der mindestens ein Rohr (26) trägt, das mit mindestens einem an ein auf dem Gerätegestell montiertes Ansaug-Rückförder-System (15) angeschlossenen Schlauch verbunden ist,
- ein mechanisches System (55 bis 70) zum Verlagern des Kopfes längs zwei Richtungen in einer horizontalen Ebene,
- Mittel (46 bis 52) zum thermischen Regulieren der Reaktionsvertie fungen (53), und
- Mittel (3, 4, 34 und 73 bis 80) für Steuerung, Kontrolle und Koor dination,
dadurch gekennzeichnet,
- daß es ferner eine mit mindestens einem Reinigungsgefäß (37) versehene Zone (12) umfaßt,
- daß die die Proben, die Reagentien, die Reaktionsvertiefungen (53), das Ablesesystem (45a) bzw. das Reinigungsgefäß (37) enthaltenden Zonen (7 bis 13) aneinander und statisch in einer einzigen, die Arbeitsebene (2) bildenden Ebene liegen,
- daß der Kopf (1) die Gesamtheit der Zonen (7 bis 13) bedient,
- und daß die Mittel (3, 4, 34 und 73 bis 80) für Steuerung, Kontrolle und Koordination derart ausgebildet sind, daß sie einerseits auf den Kopf (1) einwirken, damit dieser sich über jeder der Zonen (7 bis 13) verlagern kann, um eine Flüssigkeit aus einer Zone zu entnehmen und zu einer anderen zu transferieren oder um die Reinigung des Rohres (26) in dem Reinigungsgefäß (37) vorzunehmen, andererseits auf die thermischen Regulierungsmittel (46 bis 52) und schließlich auf das Ablesesystem (45a) einwirken.

2. Gerät nach Anspruch 1, dadurch gekennzeichnet, daß der Kopf (1) mindestens eine zweite für die Reinigung der Reaktionsvertiefungen (53) bestimmte Baugruppe (30 bis 32) umfaßt.

3. Gerät nach Anspruch 2, dadurch gekennzeichnet, daß die zweite Baugruppe einen Schlitten (30) umfaßt, auf dem mindestens ein mit mindestens einem Schlauch verbundenes Rohr (31) für das Ansaugen von in einer Reaktionsvertiefung (53) enthaltener Flüssigkeit und mindestens ein mit mindestens einem Schlauch verbundenes Rohr (32) für die Verteilung einer Lösung in einer Reaktionsvertiefung (53) befestigt sind, welche Schläuche mit dem Ansaug-Rückförder-System (15) verbunden sind.

4. Gerät nach Anspruch 3, dadurch gekennzeichnet, daß es Mittel für das Befestigen der Rohre (31, 32) an dem Schlitten (30) derart umfaßt, daß ein guter Kontakt mit dem Boden einer Reaktionsvertiefung (53) unter Kompensation eventueller Höhendifferenzen zwischen den Böden der Reaktionsvertiefungen sichergestellt ist.

5. Gerät nach Anspruch 4, dadurch gekennzeichnet, daß die Befestigungsmittel eine Feder (13) sind.

6. Gerät nach einem der Ansprüche 1 bis 5, dadurch gekennzeichnet, daß das Reinigungsgefäß (37) eine untere Öffnung (37a), über welche es entleert werden kann, und mindestens eine Flüssigkeitsinjektionsdüse (35) für die Reinigung des Äußeren des Rohres (26), wenn dieses teilweise in das Gefäß (37) eingeführt ist, umfaßt.

7. Gerät nach Anspruch 6, dadurch gekennzeichnet, daß das Reinigungsgefäß (37) ferner mindestens eine Luftinjektionsdüse (36) für das Trocknen des Äußeren des Rohres (26), wenn es teilweise in das Gefäß eingeführt ist, umfaßt.

8. Gerät nach Anspruch 1, dadurch gekennzeichnet, daß die Mittel (46 bis 52) zum thermischen Regulieren eine thermische Regulierflüssigkeit in mindestens teilweisem Kontakt mit den Vertiefungen (53) verwenden.

9. Gerät nach einem der Ansprüche 1 bis 8, dadurch gekennzeichnet, daß der Kopf (1) mindestens eine dritte Baugruppe umfaßt, gebildet von einer Kamera (34) für die Analyse der richtigen Positionierung des Kopfes und/ oder von Identifikationscodes der Proben und/oder Reagentien und das Ablesen der Analyseergebnisse.

10. Gerät nach Anspruch 9, dadurch gekennzeichnet, daß die Kamera (34) an dem Kopf (1) starr montiert ist.

11. Gerät nach Anspruch 10, dadurch gekennzeichnet, daß die Kamera (34) vertikalbeweglich an dem Kopf (1) montiert ist.

12. Gerät nach einem der Ansprüche 1 bis 11, dadurch gekennzeichnet, daß das Ablesesystem umfaßt:
- einen ersten eine Referenzflüssigkeit enthaltenden Rezipienten (45b),
- einen zweiten eine zu vermessende Probe enthaltenden Rezipienten (45a),
- eine Y-förmige optische Faser (43), die Licht zu dem ersten und zweiten Rezipienten (45b, 45a) überträgt, und
- optische Mittel (72, 44) für den Empfang des von dem ersten und dem zweiten Rezipienten kommenden Lichts.

13. Gerät nach Anspruch 12, dadurch gekennzeichnet, daß die optischen Mittel eine jeweils einem der ersten und zweiten Rezipienten (45b, 45a) zugeordnete Fotodiode oder -zelle (72, 44) umfassen.

14. Gerät nach einem der Ansprüche 1 bis 13, dadurch gekennzeichnet, daß die Mittel für Steuerung, Kontrolle und Koordination Informatik-Mittel (3, 4, 34, 73 bis 80) umfassen, ausgebildet für das Abspeichern von Analyseprotokollen, Verwalten der Identifikation von Proben, Reagentien und Reaktionsvertiefungen (53), Verwalten der verschiedenen Analyseetappen, Verwalten der Analyseergebnisse und Unterstützen des Benutzers bei Funktion und Wartung des Geräts.

## Claims

1. Device for automatic analysis of samples comprising:
- at least one head (1) capable of moving above a working plane (2) consisting in an area (7, 8) in which the samples to be analyzed are disposed, an area (9, 10) in which the reagents are disposed, an area (11) in which the reaction chambers (53) are disposed and an area (13) comprising at least one read-out system (45a) for measuring the analysis results,
- at least one first subassembly (23 to 26) for drawing up and distributing liquids, the said first subassembly being borne by the said head and comprising a carriage (26) which is capable of vertical movement and bearing at least one tube (26) which is attached to at least one pipe connected to a suction-delivery system (15) mounted on the frame of the apparatus,
- a mechanical system (55 to 70) for moving the said head along a horizontal plane in two directions,
- means (46 to 52) of thermal regulation of the said reaction chambers (53),
- and means (3, 4, 34 and 73 to 80) of control, operation and coordination,
characterized in that:
- it also comprises an area (12) equipped with at least one washing bowl (37),
- the said areas (7 to 13) comprising the samples, the reagents, the reaction chambers (53), the read-out system (45a), and the washing bowl (37) are rigidly fixed in a single plane which forms the said working plane (2),
- the said head (1) serves all the said areas (7 to 13), and
- the said means (3, 4, 34 and 73 to 80) of control, operation and coordination are designed in such a way as to act, firstly on the said head (1) so that it can move above each of the said areas (7 to 13) in order to draw up and transfer a liquid from one area to another or to wash the said tube (26) in the said washing bowl (37), secondly on said means of thermal regulation (46 to 52) and, finally, on the said read-out system (45a).

2. Device as claimed in claim 1, characterized in that the said head (1) comprises at least one second subassembly (30 to 32) used to wash the reaction chambers (53).

3. Device as claimed in claim 2, characterized in that the said second subassembly comprises a carriage (30) on which is fixed at least one tube (31) connected to at least one pipe, in order to draw up the liquid contained in a reaction chamber (53) and at least one tube (32) connected to at least one pipe, in order to distribute a solution in a reaction chamber (53), the said pipes being connected to the said suction-delivery system (15).

4. Device as claimed in claim 3, characterized in that it comprises means of fixing the said tubes (31, 32) to the said carriage (30) in such a way as to ensure a close contact with the bottom of a reaction chamber (53), whilst compensating for any differences in height between the bottom of each of the said reaction chambers.

5. Device as claimed in claim 4, characterized in that said fixing means are in the form of a spring (13).

6. Device as claimed in any of the claims 1 to 5, characterized in that the said washing bowl (37) comprises a lower aperture (37a) by which it may be emptied and at least one injection nozzle (35) for introducing the liquid used to clean the exterior of the said tube (26) when this is partially inserted into the said bowl (37).

7. Device as claimed in claim 6, characterized in that the said washing bowl (37) also comprises at least one air injection nozzle (36) for drying the exterior of the said tube (26) when this tube is partially inserted into the bowl.

8. Device as claimed in claim 1, characterized in that said means (46 to 52) of thermal regulation use a thermal regulation liquid which is in at least partial contact with the said chambers (53).

9. Device as claimed in any of the claims 1 to 8, characterized in that the said head (1) comprises at least a third subassembly formed from a camera (34) to verify that the head is correctly positioned and/or to check the identification codes of the samples and/or the reagents and to read the analysis results.

10. Device as claimed in claim 9, characterized in that the said camera (34) is rigidly fixed onto the said head (1).

11. Device as claimed in claim 10, characterized in that the said camera (34) is mounted in such a way that it is capable of vertical movement on the said head (1).

12. Device as claimed in any of the claims 1 to 11, characterized in that the said read-out system comprises:
- a first recipient (45b) containing a reference liquid;
- a second recipient (45a) containing a sample to be measured;
- an optical fibre (43) in the form of a Y, which sends light to the said first and second recipients (45b, 45a); and
- optical means (72, 44) of receiving the light coming from the said first and second recipients.

13. Device as claimed in claim 12, characterized in that said optical means comprise a photodiode or cell (72, 44) connected to each of the said first and second recipients (45b, 45a).

14. Device as claimed in any of the claims 1 to 13, characterized in that said means of control, operation and coordination comprises data processing means (3, 4, 34, 73 to 80), capable of memorizing the protocols of the analyses, managing identification of the samples, the reagents and the reaction chambers (53), managing the various stages of the analyses, managing the results of the analyses and assisting the user in the operation and maintenance of the device.
